# EUROPEAN PATENT APPLICATION

(11) **EP 2 048 129 A1**
(43) Date of publication of application: **15.04.2009**
(21) Application number: 07020022.5
(22) Date of filing: 12.10.2007
(51) Int. Cl.: C07C 201/02, C07C 201/12, C07C 203/04, C07C 205/57

(54) **Method for the preparation of organic nitrates**

(71) Applicant: Lonza AG, 4052 Basel (CH)
(72) Inventor: The designation of the inventor has not yet been filed

(57) **Abstract**

The present invention relates to a process for the preparation of organic nitrates having at least one nitryloxy and at least one hydroxy group, wherein the at least one hydroxy group may be present in form of an esterified hydroxy residue, the latter being esterified with an acid other than nitric acid.

## Description

The present invention relates to a process for the preparation of organic nitrates having at least one nitryloxy and at least one free hydroxy group or esterified hydroxy residue. Here and hereinbelow the term "esterified hydroxy residue" represents a hydroxy group esterified with an acid or an acid anhydride other than nitric acid. Hydroxy groups esterified with nitric acid are referred to as nitryloxy groups.

Organic nitrates having at least one nitryloxy group (esters of an alcohol with nitric acid) and at least one free hydroxy group are valuable building blocks. Several pharmaceutically active compounds contain nitryloxy groups which stimulate the endogenous production of nitric oxide and/or are substrates for nitric oxide synthase and/or cytochrome P450 by releasing nitric oxide (nitric oxide donors). An example for the combination of anti-inflammatory agents like 2-(*S*)-(6-methoxy-2-naphthyl)-propanoic acid (naproxene) with one of the above mentioned building blocks in the CINOD (COX-inhibiting-nitric oxide-donating) class is Naproxcinod 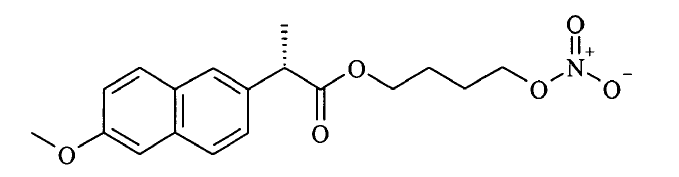 disclosed for example in WO-A 95/30641, WO-A 98/25918 and WO-A 01/10814.

Unfortunately, the preparation of organic nitrates (especially nitrates with more than one nitryloxy group, like glycerol trinitrate) is connected with safety issues since organic nitrates are explosive and therefore difficult to handle even in diluted solution. In addition the use of highly corrosive acids and the need of management of large amounts aqueous nitrate waste render the industrial production of organic nitrates difficult.

EP-A 0 359 335 discloses 6-acyloxy-1-hexanol nitrate among several alcohol nitrates, diol dinitrates and glyceryl trinitrate. 6-Acetyloxy-1-hexanol nitrate has been extracted from the reaction mixture obtained by nitration of 1 equivalent (eq.) 1,6-hexanediol with 2.2 eq. nitric acid in the presence of acetic acid.

EP-A 0 361 888 discloses the preparation of heterocyclic esters of ω-nitryloxy-alkan-1-ols having a straight C₂₋₁₁ alkylene chain, particularly 3-nitryloxy-*n*-propanol, 6-nitryloxy-*n*-hexanol and 11-nitryloxy-*n*-undecanol by reacting the respective ω-bromo-alkan-1-ol with AgNO₃.

WO-A 94/021248 discloses a method for the preparation of 5-acetoxy-1-pentanol nitrate and 9-acetoxy-1-nonanol nitrate by reacting 1 equivalent (1 eq.) of the respective diol with 4 eq. acetic anhydride and 2 eq. nitric acid. For exhaustive nitration of alcohols having 1 to 3 hydroxy groups the respective alcohol is reacted with 3 eq. acetic anhydride and 3 eq. nitric acid per hydroxy group. After extraction of the crude mixture the products have been separated by chromatography or crystallization. No data regarding purity, yield and efforts for work-up are given. A major disadvantage of this reaction is that acetyl nitrate formed in the reaction from acetic anhydride and nitric acid is a highly explosive compound.

WO-A 98/25918 discloses the preparation of aralkyl esters of nitrated cycloaliphatic diols by reacting an aralkylic acid derivative with the respective mononitrated cycloaliphatic diol. The alcohols are prepared by nitration of the respective monohalogenated cycloaliphatic diol or by reacting the respective diol with acetic anhydride and nitric acid. No yields are given. The existence of the respective acetoxy derivative is not reported.

WO-A 01/10814 discloses an improved method over WO-A 98/25918 regarding the preparation of 4-nitryloxybutyl 2-(6-methoxy-2-naphthyl)-propanoate wherein 4-nitryloxy-butan-1-ol (1,4-butanediol mononitrate, BDMN) is prepared according to methods known in the art.

WO-A 2004/012659 discloses the nitration of 2-bromoethanol, 3-bromopropan-1-ol, 5-bromopentan-1-ol and 6-bromohexan-1-ol with AgNO₃ according to methods known in the art, and the subsequent esterification of the alcohol with phosgene to obtain the respective chloroformates. It also discloses the preparation of a mixture of 3-nitryloxy-2-(nitryloxy-methyl)propan-1-ol and 2-(nitryloxymethyl)propane-1,3-diol by reacting 2-(hydroxymethyl)-1,3-propanediol with a mixture of acetic anhydride and nitric acid.

WO-A 2004/043897 discloses a purification method of BDMN from mixtures obtained by nitration of 1,4-butanediol with "stabilized" nitric acid according to the method also disclosed in WO-A 2004/043898.

WO-A 2004/043898 discloses the preparation of ω-nitryloxy-alkane-1-ols having a straight or branched C₂₋₆ alkylene chain by reacting the respective diol with "stabilized" nitric acid. Reported yields are about 37%. The crude product is subject to unspecified "subsequent purification".

WO-A 2006/006375 discloses the preparation of 6-nitryloxy-hexan-1-ol and 4-nitryloxy-butan-1-ol and as building blocks by reacting 6-bromo-hexan-1-ol with AgNO₃ and by reacting 4-bromobutyl acetate with AgNO₃ followed by hydrolysis of the 4-acetoxybutyl nitrate, respectively.

A persistent aim of the chemical industry is to constantly improve and control chemical reactions. Greater control over reactions may lead to, for example, improvements in safety, increase in reaction product yield and/or purity, or the isolation of valuable highly reactive intermediate products. In particular, greater control over reagent mixing, fluid flow, heat sinking/sourcing and catalytic efficiency is desirable.

A general method which provides such improved control over reactions would therefore be advantageous. Particularly, methods for performing exothermic reactions and/or reactions forming explosive compounds in large scale in a controlled manner are sought for.

The technical problem to be solved by the present invention was to provide an alternative method for the preparation of nitryloxy alcohols starting from compounds having at least two hydroxy groups and wherein at least one hydroxy group shall be maintained after nitration either as a free hydroxy group or masked as an ester. A further problem to be solved was to establish said process in a robust and secure manner. Furthermore side reactions should be avoided in order to simplify the work-up procedures. In addition, the general concept should start with easily available compounds and should contain few reaction steps.

The problem could be solved according to the process of claim 1.

Provided is a method for the preparation method for preparation of a compound having at least one nitryloxy group and at least one hydroxy group or an esterified hydroxy residue, said esterified hydroxy group representing a hydroxy group esterified with an acid other than nitric acid and thus consisting of an acid moiety and oxygen, comprising reaction of a compound comprising at least one esterified hydroxy group and a least one free hydroxy group with nitric acid, optionally in the presence of another acid or acid anhydride, to obtain a compound
wherein the at least one free hydroxy group is exhaustively nitrated, and, optionally, subsequent hydrolysis or transesterification of the at least one esterified hydroxy residue.

Surprisingly, the esterified hydroxy residue is stable enough under nitration reaction conditions.

By direct (non-exhaustive) nitration of only one or more (but less than maximum) hydroxy groups of a compound having at least two hydroxy groups a mixture of non-, mono- and polynitrated compounds are obtained. Extraction and/or separation (work-up) of the products from the starting material is difficult because the exchange of a hydroxy group by a nitro group is not connected with a strong change in polarity. Furthermore, concentration and especially distillation of organic nitrates is extremely hazardous.

Compared to nitrations known in the art of compounds having for example two free hydroxy groups to afford compounds having one free hydroxy and one nitryloxy group which can be carried out in at concentrations of about maximum 5% regarding the compound to be nitrated advantageously the instant process can be carried out in concentrations up to 15% because over-nitration is avoided. In contrast to processes known in the art the instant process aims towards exhaustive nitration of all free hydroxy groups. Hydroxy groups which should be maintained for further reactions are masked as esters.

Here and hereinbelow the term "compound comprising at least one esterified hydroxy group and a least one free hydroxy group" generally represents an alcohol moiety having a di- or polyol main structure wherein at least one of the hydroxy groups is esterified with an acid moiety.

Here and hereinbelow the term "di- or polyol main structure" represents the free alcohol which is obtained after hydrolysis of the starting compound. Due to the various possibilties of pretecting and esterificating di- and polyols it is not suitable to mention the suitable di- or polyol moiety itself but said corresponding main structure.

Suitable diol main structures comprise for example C₂₋₁₈-diols such as ethanediol, 1,2-propanediol, 1,3-propanediol, 1,2-butanediol, 1,3-butanediol, 1,4-butanediol, 2,3-butanediol, pentanediols or hexanediols. Preferably the diol main structure is 1,4-butanediol. Suitable polyol main structures comprise for example glycerol, carbohydrates and derivatives such as free or protected aldoeses and ketoses. Aldoses and ketoses might be protected for example by reacting with acetone in form of their ketals. Preferably the carbohydrates are pentoses or hexoses such as aldo- and ketopentoses and aldo- and ketohexoses and derivatives thereof. Examples of suitable pentoses and hexoses are each D- and L-lyxose, -xylose, -arabinose, -ribose, -deoxyribose, -ribulose, -allose, -altrose, -glucose, -mannose, -gulose, -idose, -galactose, -talose, -psicose, -fructose, -sorbose and -tagatose as well in their respective open chain, pyranose (cyclic hemiacetal of an aldohexose) and furanose (cyclic hemiacetal of an aldopentose or a cyclic hemiketal of a ketohexose) ring forms or after protection for example by reacting with acetone. Preferably the polyol is selected from the group consisting of glycerol, D- and L-arabinose, -deoxyribose, -ribulose, -glucose, -mannose, -galactose, -fructose, and -sorbose.

Also surprisingly, side-products from acetylation are removed during ester hydrolysis. Especially for short chain and/or polar di- and polyol having 2 to about 6 carbon atoms such as propanediols, butanediols, pentanediols, hexanediols, glycerol, pentoses or hexoses side-products from esterification can be removed during work-up after hydrolysis. Thus the whole process is suitable for production on industrial scale.

Here and hereinbelow the term "acid moiety" which is part of the esterified hydroxy residue consists of a functional group comprising a hetero atom-carbon or a hetero atom-sulfur double bond such as carbonyl (-CO-), thiocarbonyl (-CS-), sulfinyl (-SO-) or sulfonyl (-SO₂-) which is attached to oxygen and having also attached a further residue.

In one embodiment said further residue is an alkyl or aryl group forming an acyl, aroyl, alkanesulfonyl and arenesulfonyl group.

Thus, in another embodiment said further residue is attached via an oxygen, nitrogen or sulfur atom such as HO(CH₂)ₘO-, R³R⁴N- or R⁵S- groups, forming for example a carbonic acid diester, a carbamate or a thioester, wherein R³, R⁴ and R⁵ independently are selected from the group consisting of C₁₋₁₈-alkyl and aryl groups as defined above, and wherein m is an integer from 2 to 18.

Preferably the acid moiety of the at least one esterified hydroxy residue is selected from acyl, aroyl, alkanesulfonyl and arenesulfonyl groups, said acyl or alkanesulfonyl groups consisting of an alkyl moiety and a carbonyl or sulfonyl group respectively, said alkyl moiety being optionally further substituted with one or more halogen atoms, and said aroyl and arenesulfonyl groups consisting of an aryl moiety and a carbonyl or sulfonyl group respectively, said aryl moiety being optionally further substituted with one or more halogen atoms and/or one or more nitro, alkyl, alkoxy, aryl and aryloxy groups. Some aromatic moieties of the acid moiety may be subject to nitration also, thus requiring more equivalents nitric acid without limiting the general advantages of the inventive process.

Here and hereinbelow the term "alkyl" represents a linear or branched alkyl group. By using the form "C₁₋ₙ-alkyl" the alkyl group is meant to have 1 to n carbon atoms. C₁₋₆-alkyl represents for example methyl, ethyl, propyl, isopropyl, butyl, isobutyl, *sec*-butyl, *tert*-butyl, pentyl and hexyl.

Here and hereinbelow the term "alkoxy" represents a linear or branched alkoxy group. By using the form "C₁₋ₙ-alkoxy" the alkyl group is meant having 1 to n carbon atoms. C₁₋₆-alkoxy represents for example methoxy, ethoxy, propoxy, isopropoxy, butoxy, isobutoxy, *sec*-butoxy, *tert*-butoxy, pentyloxy and hexyloxy.

Here and hereinbelow the term "aryl" represents an aromatic mono, di- or tricyclic group having 6 to 14 carbon atoms, optionally comprising 1 to 4 ring heteroatoms selected from the group consisting of nitrogen, oxygen and sulfur. Optionally the aryl moiety is further substituted as defined in the specific case.

Preferably "aryl" means phenyl or naphthyl optionally being further substituted as mentioned above for aryl.

Here and hereinbelow the term "aryloxy" represents an aromatic mono, di- or tricyclic group having 6 to 14 carbon atoms directly bound via an ether oxygen atom, optionally comprising 1 to 4 ring heteroatoms selected from the group consisting of nitrogen, oxygen and sulfur. Optionally the aryl moiety is further substituted as defined in the specific case.

Preferably "aryloxy" means phenoxy or naphthoxy optionally being further substituted as mentioned above for aryloxy.

Here and hereinbelow the term "aralkyl", represents a C₁₋₈ alkyl group as defined above, substituted with an aryl moiety as defined above.

Here and hereinbelow the term "alkanesulfonyl", represents a C₁₋₈ alkyl group as defined above, substituted with a sulfonyl moiety. An example for alkanesulfonyl with a C₁ alkyl group is the methanesulfonyl (mesyl) residue.

Here and hereinbelow the term "arenesulfonyl", represents an aryl group as defined above, substituted with a sulfonyl moiety. An example for arenesulfonyl with a tolyl moiety is the 4-toluenesulfonyl (tosyl) residue.

Here and hereinbelow the term "halogen atom", represents a fluorine, chlorine, bromine or iodine atom, preferably a chlorine or bromine atom.

In a preferred embodiment the inventive process is carried out by nitration of a compound of the formula

R¹C(O)O(CH₂)ₙOH (I)

wherein R¹ is selected from the group consisting of C₁₋₁₈-alkyl and aryl, and wherein n is an integer from 2 to 18, to obtain a compound of the formula

R¹C(O)O(CH₂)ₙONO₂ (II)

wherein R¹ and n are as defined above.

Thus, taking in account the above a special case arises wherein one hydroxy of two equivalents of the starting compound is esterified with a carbonic acid derivative. Thus, after hydrolysis or transesterification of the nitrated product CO₂ and two equivalents of a nitrated compound having at least one nitroxy group and at least one hydroxy group or esterified hydroxy group will be released. In an alternative process two hydroxy groups of a starting compound having at least three hydroxy groups are protected from nitration by forming a cyclic carbonic acid diester. Thus, among CO₂ only one equivalent of a nitrated compound having at least one nitroxy group and at least two hydroxy groups or esterified hydroxy groups will be released after hydrolysis or transesterification.

In a further preferred embodiment the inventive process is carried out by nitration of a compound of formula

R²C(O)O(CH₂)ₙOH (III)

wherein R² is a halogen atom or a residue selected from the group consisting of O₂NO(CH₂)ₘO-, R³R⁴N- and R⁵S-, wherein R³, R⁴ and R⁵ independently are selected from the group consisting of C₁₋₁₈-alkyl and aryl groups as defined above, and wherein n and m independently are an integer from 2 to 18, to obtain a compound of the formula

R²C(O)O(CH₂)ₙONO₂ (IV)

wherein R², R³, R⁴, R⁵, n and m are as defined above. In another preferred embodiment exhaustive nitration of a compound of the formula

HO(CH₂)ₘOC(O)O(CH₂)ₙOH (IIIa),

wherein n and m are the same is carried out. Said compound of formula IIIa could be prepared for example by esterification of phosgene with 2 equivalents of the respective diol. Where n = m = 4, hydrolysis of the dinitrate will release CO₂ and 2 eq. BDMN. Hydrolysis and transesterification are suitable alternatives in the main process. The ester group might also be subject to a process to exchange the ester group which has been used to carry out the nitration of the free hydroxy group(s) with another ester group. Transesterification can be carried out as well as known in the art.

The hydrolysis may be carried out chemically or enzymatically. Chemical ester hydrolysis may be carried out in the presence of a base or acid catalyst. Preferably the catalyst is a base such as alkaline metal or earth alkaline metal hydroxide, alkaline metal or alkaline earth metal alkoxide, trimethylamine, triethylamine, ammonia and mixtures thereof. A suitable acidic catalyst might be selected from the group consisting of organic or inorganic acids, preferably of non-oxidizing acids. Examples for suitable organic acids are formic acid, acetic acid or propionic acid. Examples for suitable inorganic acids are HCl, HF or solid acids like montmorillonite or zeolithes.

Ester cleaving enzymes belong to the "hydrolase" group having an EC 3 classification, more specifically they belong to the "esterase" group, having an EC 3.1 classification. The enzymes might be used as known in the art in their native form using microorganisms having a hydrolases and/or esterase activity, as extracts thereof or in more or less purified form.

In a further preferred embodiment the compound comprising at least one esterified hydroxy residue and a least one free hydroxy group is obtained by esterification of a compound having at least two free hydroxy groups. In the art methods are known for selective partial esterification.

In a further preferred embodiment the compound comprising at least one esterified hydroxy residue and a least one hydroxy group is obtained by partial ester hydrolysis of a compound having at least two esterified hydroxy residues. The starting compound can be easily prepared from a diol or polyol by reacting with a carboxylic or sulfonic acid halogenide or anhydride or even may be commercially purchased.

The nitrification may be carried out in the presence of another acid or acid anhydride beside nitric acid, wherein said another acid may support the nitrification. Suitable other acids or anhydrides are for example sulfuric acid, methanesulfonic acid, toluenesulfonic acid, alkanoic anhydrides or aroyl anhydrides. Examples for alkanoic and aroyl anhydrides are acetic anhydride or benzoic anhydride. Some aromatic aids may also be subject to nitration, thus requiring more equivalents of nitric acid, without limiting the general advantages of the inventive process.

According to the present invention there is also provided a method for the preparation of a compound having at least one nitryloxy group and at least one hydroxy group or an esterified hydroxy residue, said esterified hydroxy group representing a hydroxy group esterified with an acid other than nitric acid and thus consisting of an acid moiety and oxygen, comprising reaction of a compound comprising at least one esterified hydroxy group and a least one free hydroxy group with nitric acid in a microreactor (MR), optionally in the presence of another acid or acid anhydride, to obtain a compound wherein the at least one free hydroxy group is exhaustively nitrated, and, optionally, subsequent hydrolysis or transesterification of the at least one esterified hydroxy residue

In a preferred mode of action said nitration reaction comprising mixing at least two fluids,
wherein one of the at least two fluids comprising a compound having at least one ester and at least one nitro group (1^{st} reactant), and another fluid comprising nitric acid, optionally in the presence of another acid (2^{nd} reactant), and optionally further fluids, said mixing taking place in a microreactor (6) comprising at least one flow path (1) for one of the at least two fluids (A) comprising either the 1^{st} or 2^{nd} reactant, said flow path(s) comprising at least one reaction region (2), each reaction region comprising an injection point (3) for feeding the other one of the at least two fluids (B) comprising either the 2^{nd} or the 1^{st} reactant, a mixing zone (4) in which the at least two fluids contact each other and a reaction zone (5), and wherein the microreactor optionally provides one or more additional residence time volumes, and wherein in said method one of the at least two fluids comprising either the 1^{st} or 2^{nd} reactant establishes a first flow and wherein the other one of the at least two fluids comprising either the 2^{nd} or 1^{st} reactant is injected into said first flow at least at two injection points (3) along said flow path(s) (1) in a way such that at each injection point only a fraction of the amount necessary to reach complete hydroxy group nitration is injected.

Usually the expression "microreactor" is used for a reactor which reaction volumes have dimensions (perpendicular to the flow direction) of about 10000 micrometers and less.

The expression "necessary to reach complete hydroxy group nitration" means the amount which would have to be added to reach "theoretical" completion of the reaction for example in a single vessel. In a simple 1:1 reaction stoichiometry this would be equimolar amounts. For a 1^{st} reactant like monoacetyl glycerol at least two molar equivalents of a nitric acid is necessary to complete the reaction of double nitration. Diacetyl glycerol would require at least one molar equivalent to reach complete nitration.

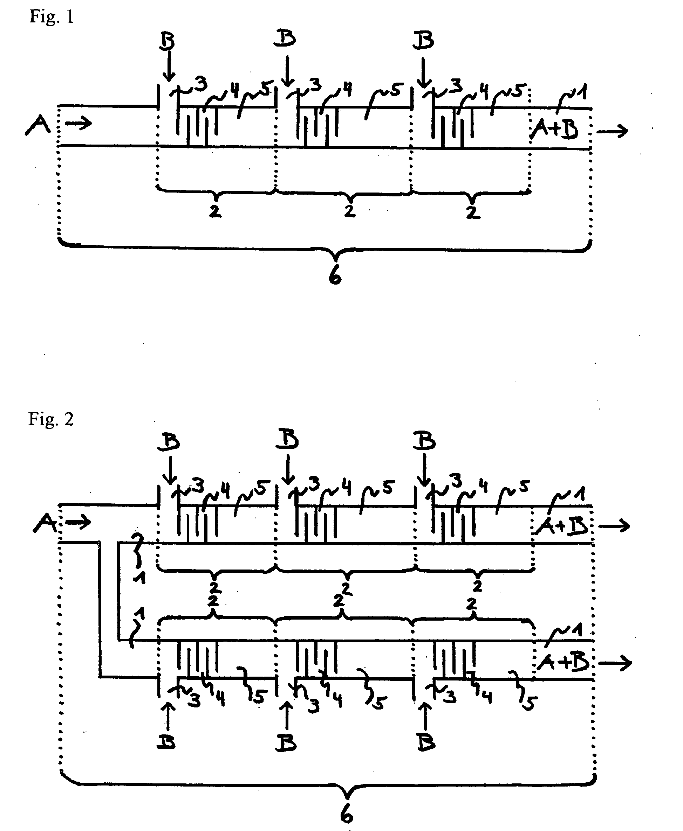

Fig. 1 and Fig. 2 show two examples of feeding a flow B at various injection points to a flow A. The microreactor (6) in Fig. 1 comprises one flow path with three injection points, the microreactor (6) in Fig. 2 comprises two flow paths each having three injection points. There may be more than two flow paths present, as well as more than three injection points in each flow path. Thus, the 2^{nd} reactant may be fed at the injections points to a first flow generated by the fluid comprising the 1^{st} reactant.

Furthermore, there are no structural limits regarding the injection points, the mixing zones and/or the reaction zones. Only for the reason of better understanding of the parts of the microreactor used in the present invention the microreactors in Fig. 1 and Fig. 2 are depicted as a linear strung-out hollow space. Nevertheless, the flow path(s) (1) may be tortuously bent as known in the art. Furthermore, different mixing zones and/or reaction zones need not have the same dimensions in width or length. It is further not necessary to use a microreactor which contains all of the features mentioned above in one physical entity. It is also possible to externally connect additional injection points, mixing zones, reaction zones, each optionally cooled or heated, to a flow path.

Feeding only a fraction of the amount necessary to reach completion of the nitration reaction while using more than one mixing zone and/or injection point leads to an increase the number of hot spots in the microreactor while the temperature rise in each hot spot is reduced as compared to typical micro reactors with only one mixing and reaction zone. In addition, since one of the two compounds is diluted in the first flow comprising the other compound, formation of side products is reduced and yields are increased. Thus, the inventive method provides an improved control over reactions.

In the present invention depending on the mixing properties of the mixing zone it is not necessary that the at least two fluids are completely miscible.

In addition, to the at least one general flow path, at least one injection point, at least one mixing zone and at least one reaction zone a suitable microreactor for the inventive method may comprise additional structural elements such as temperature adjustable retention volumes, temperature adjustable premixing volumes and others known in the art.

It has been found that using a microreactor is particularly advantageous for nitration reactions if used with multiple-injection points and/or multiple mixing zones. According to the present method, improved control over a batch nitration reaction can be achieved, which can result in significant improvements in reaction product yield and/or purity, as well as other benefits. The reaction starts after contacting the reactive fluids A and B in the mixing zone (3) and continues in a reaction zone (3). In a preferred embodiment the flow path(s) (1) has/have a width in the range of 10 to 10000 micrometers and a cross section of 0.1 square centimeters or less. More preferably the flow path width is in a range of 10 to 500 micrometers, or even more preferably in a range of 10 to 200 micrometers.

In a further preferred embodiment heat or cooling independently is supplied to the reservoirs of reactants, injection point(s) (3), the mixing zone(s) (4) and/or the reaction zone(s) (5) or any other structural entity of the microreactor used. Preferably the heat or cooling is supplied by an external source. Said heat or cooling can be supplied to initiate, maintain and/or slow down the reaction. Preferably heat is supplied to initiate and/or maintain the reaction, whereas cooling is supplied to slow down the reaction. In rare cases heat may be supplied to slow down the reaction, whereas cooling may be supplied to initiate and/or maintain the reaction.

Generally, the first flow (1) of fluids containing the reaction product is quenched after being discharged from the microreactor. Fast exothermic reactions which are almost completed when the reaction mixture has passed the mixing zone may require additional cooling while passing the reaction zone to suppress side product formation. Performing slow reactions to complete conversion often leads to side products. In a preferred embodiment the product is isolated after quenching the reaction. In case the reaction does not reach completion in the mixing zone for several nitration reactions it may be suitable to feed the discharged first flow from the reaction zone or the microreactor into an external retention volume for further reaction, for other nitration reactions it may be suitable after the last injection point to quench the first flow directly after being discharged from the reaction zone or from the microreactor before it reaches completion to avoid excessive formation of oxidative side-products.

The temperature and thus the selectivity of the nitration reaction can be easily controlled with an increasing number of mixing zones and/or injection points. Comparing the benefit of each additional injection zone with the efforts and drawbacks of connecting or building a further injection zone (new microreactor design, in general increase of required hardware, additional programming work, increased fluid pressure, increased danger of leakage) it has been found, that the inventive method is advantageously carried out with a microreactor comprising not more than 7 reaction regions (injection points, mixing zones, reaction zones), preferably 3 to 6 reaction regions. Most preferably the mixing zones above one can be used independently of the injection points.

Suitable microreactors (MR) to be used in the instant process could be made of different materials (glass or metal) and may belong to different built systems provided they are built for reactions under corrosive conditions. Integrated microreactor entities wherein injection point(s), mixing zone(s) and reaction zone(s) are built in one physical entity or MR which are made from single elements (injection point(s), mixing zone(s) and reaction zone(s)) connected via external fittings are both suitable. The same applies to MR were temperature is adjusted by immersing in a temperature controlled bath without having any additional elaborated temperature adjustment systems in place or MR comprising an efficient internal temperature adjustment system wherein a temperature controlled fluid is fed to the outside surface of injection point(s), mixing zone(s) and reaction zone(s) to provide an efficient and quick temperature adjustment. The starting compound and the nitration reagent each can be fed in one or more inlet points.

Provided are nitrates obtainable by the instant process comprising at least one nitryloxy group and at least one esterified hydroxy residue, comprising at least one acid moiety as mentioned above and at least one di- or polyol main structure mentioned above with the proviso of compounds wherein the acid moiety is acetate and the diol main structure is a linear C₂₋₁₈-diol such as 4-acetoxy-1-butyl nitrate, 6-acetoxy-1-hexyl nitrate or 11-acetoxy-11-undecyl nitrate.

Also provided are compounds of the formula

R¹C(O)O(CH₂)ₙONO₂ (IIa)

wherein R¹ is selected from the group consisting of C₂₋₁₈ alkyl and aryl, and wherein n is an integer from 2 to 18.

Additionally provided are carbonic acid diesters (carbonates) wherein two, optionally identical, residues are attached to a central carbonyl group via an oxygen atom and wherein each of the residues having at least one free hydroxy group. Consequently, provided are also carbonic acid diesters wherein two, optionally identical, residues are attached to a central carbonyl group via an oxygen atom and wherein each of the residues having at least one nitryloxy group. Carbonates wherein the residues are not identical can be obtained by reacting a halogen carbonic acid monoester with a compound having at least two free hydroxy groups which is different from the residue already attached to the central carbonyl group. Said halogen carbonic acid monoesters are obtainable to methods known in the art by reacting phosgene with a compound having at least two free hydroxy groups.

Furthermore, provided are cyclic carbonic acid diesters wherein one residue is attached via two oxygen atoms to a central carbonyl group and the residue comprises at least one free hydroxy group. Additionally provided are, cyclic carbonic acid diesters wherein one residue is attached via two oxygen atoms to a central carbonyl group and wherein the residue comprises at least one nitryloxy group.

More preferably provided are compounds of the formula

R²C(O)O(CH₂)ₙONO₂ (IVa)

wherein R² is a halogen atom or a residue selected from the group consisting of O₂NO(CH₂)ₘO-, R³R⁴N- and R⁵S-, wherein R³, R⁴ and R⁵ independently are selected from the group consisting of C₁₋₁₈-alkyl and aryl groups as defined above, and wherein n and m independently are an integer from 2 to 18, with the exception of compounds wherein R² is chlorine and n is an integer from 2 to 6. These compounds have been obtained in WO-A 2004/012659 by esterification of the corresponding nitryloxy alcohol with about threefold molar excess of phosgene.

Further objects, advantages and features may be derived from the dependent claims and the described embodiments of the present invention.

### Examples

### Example 1: Preparation of 1,4-butanediol monoacetate with liquid catalyst

A 10-L glass vessel equipped with an impeller was charged with 1,4-butanediol (400 g, 4.4 mol), acetic acid (254.0 g, 4.2 mol) and CH₂Cl₂ (4.0 kg) at 20 °C. To the clear solution, 98% sulfuric acid (21.2 g, 0.22 mol) was added dropwise over 5 min and the resulting cloudy mixture was stirred at 20 °C for 6 hr. The reaction mixture was extracted with aqueous 5% Na₂CO₃ solution (2×1300 mL) to remove unreacted acetic acid. The organic phase was then washed with water (2×500 mL). After removal of the solvent *in vacuo* (35 °C, 600 mbar) a liquid product (289.3 g) was obtained. GC analysis (FID): 1,4-butanediol monoacetate 91.8%-area.

### Example 2: Preparation of 1,4-butanediol monoacetate with solid catalyst

A 10-L glass vessel equipped with an impeller and reflux condenser was charged with 1,4-butanediol (240.1 g, 2.7 mol), acetic acid (152.0 g, 2.5 mol), CH₂Cl₂ (1.2 kg) at 20 °C and amberlyst 36 (12.0 g). The resulting clear mixture was heated to reflux. After 24 h, the mixture was cooled, the solid catalyst filtered off and the organic phase washed with aqueous 5% Na₂CO₃ solution (2×400 mL) and water (2×170 mL). The solvent was removed *in vacuo* affording 179.4 g of a clear liquid. According to GC analysis (FID) 1,4-butanediol monoacetate was obtained in 92.0 % yield.

### Example 3: Preparation of 4-nitrobenzoic acid 4-hydroxybutyl ester

Under nitrogen atmosphere 1,4-butane-diol (90.0 g, 1.0 mol, 3.7 eq.), 4-(dimethylamino)-pyridine (DMAP, 0.3 g), acetone (300 mL) and triethylamine (23.4 g) were added successively and cooled to -5 °C. A mixture of *p*-nitrobenzoyl chloride (37.1 g, 0.27 mol, 1 eq.) in acetone (200 mL) was added drop-wise while maintaining the inner temperature at <0 °C. After complete addition, the obtained mixture was stirred at 0 °C for 1 h. Then the solvent was removed at reduced pressure, CH₂Cl₂ (500 mL) was added to the residue and the resulting and the mixture was washed with brine (3×300 mL). The organic residue was dried by Na₂SO₄, filtered and the solvent was removed at reduced pressure until dryness. The product 4-nitrobenzoic acid 4-hydroxybutyl ester was obtained as yellow solid (31.5 g, 66%).

### Examples 4 and 5: 3-nitrobenzoic acid 4-hydroxybutyl ester and 3-chlorobenzoic acid 4-hydroxybutyl ester

According to example 3, 3-nitrobenzoic acid 4-hydroxybutyl ester and 3-chlorobenzoic acid 4-hydroxybutyl ester have been prepared by reacting 1,4-butanediol with 3-nitrobenzoyl chloride or 3-chlorobenzoyl chloride, respectively.

### Example 6: 4-nitrobenzoic acid 4-nitryloxybutyl ester in batch reaction

Conc. H₂SO₄ (98%, 100 mL) was charged in a vessel and then cooled to 0 °C. Conc. HNO₃ (65%, 50 mL) was added drop-wise. 4-Nitrobenzoic acid 4-hydroxybutyl ester (25 g) was charged in one portion, then stirred at 0 °C for 1 h. The reaction mixture was poured on ice-cold water (500 mL), then extracted by CH₂Cl₂ (2×300 mL). The mixture of combined organic layers was washed with brine (200 mL), dried over MgSO₄ and filtered. After removal of the solvent the product 4-nitrobenzoic acid 4-nitryloxybutyl ester (27.9 g, 94%) was obtained a light yellow oil which after several days became a white solid.

### Example 7: 3-nitrobenzoic acid 4-nitryloxybutyl ester

According to example 6, 3-nitrobenzoic acid 4-nitryloxybutyl ester has been prepared with 94% yield by nitration of 3-nitrobenzoyl chloride.

### Example 8: 3-chloro-4-nitrobenzoic acid 4-nitryloxybutyl ester

According to example 6, by nitration of 3-chlorobenzoic acid 4-hydroxybutyl ester 3-chloro-4-nitrobenzoic acid 4-nitryloxybutyl ester has been prepared with 91 % yield. In this case a double nitration (i.e. at the hydroxy group and at the aromatic acid moiety) took place in excellent purity.

### Example 9: Hydrolysis of acetic acid 4-nitryloxybutyl ester

A 500 mL three-necked flask was charged under nitrogen at 20 °C acetic acid 4-nitryloxybutyl ester (250.1 g of a 5.0 wt% solution in CH₂Cl₂, 12.5 g neat, 70.6 mmol), then the CH₂Cl₂ was distilled off at a temperature of 24 to 28 °C first under moderate vacuum (about 400 mbar), and under accelerated vacuum (about 20 mbar) toward the end of the distillation. During the distillation, the distilled CH₂Cl₂ was continuously replaced by water (total quantity: 237.5 g) in order to have a constant volume. When the distillation was finished, the obtained emulsion was cooled to 0 °C and 2 M sodium hydroxide solution (71 mL) was added in one portion. After the addition, the resulting mixture was stirred for 90 minutes at 0 °C affording a yellow solution. When the reaction was complete (GC control), CH₂Cl₂ (75 mL) was added to the reaction mixture. After 10 minutes stirring, the agitation was stopped, the two phases were separated and the aqueous phase was then extracted with CH₂Cl₂ (2×75 mL). The collected organic phases were dried over sodium sulfate and filtrated affording the product 4-nitryloxybutan-1-ol (1,4-butanediol mononitrate, BDMN) as a colourless solution in CH₂Cl₂ (196.7 g). The assay of the product in the solution was approximately 4.3 wt%, thus according to ¹H-NMR corresponding to approx. 90% yield.

### Example 10: Hydrolysis of 4-nitrobenzoic acid 4-nitryloxybutyl ester

4-Nitrobenzoic acid 4-nitryloxybutyl ester (2.84 g, 10 mmol) was dissolved in THF (20 mL) and charged with water (10 mL). After cooling to 0 °C 1 M aqueous NaOH (10 mL) was added drop-wise. After the addition, the mixture was stirred another 1 h at 0 °C. THF was removed from the reaction mixture by reducing pressure. Then the solution was extracted with CH₂Cl₂ (3×20 mL). The mixture of the combined organic layers was washed with brine (20 mL) and dried over MgSO₄. After filtration and removing the solvent BDMN has been obtained as yellow oil (1.26 g, 94%).

### Examples 11 and 12: Hydrolysis of 3-nitrobenzoic acid 4-nitryloxybutyl ester and 3-chloro-4-nitrobenzoic acid 4-nitryloxybutyl ester

According to example 10, hydrolysis of 3-nitrobenzoic acid 4-nitryloxybutyl ester and 3-chloro-4-nitrobenzoic acid 4-nitryloxybutyl ester have been carried out. In both cases BDMN was obtained in 94% and 90% yields, respectively.

### Example 13: Preparation of acetic acid 4-nitryloxybutyl ester in a microreactor

Two stock solutions were prepared for reaction in a microreactor (MR). Feed-1 solution was prepared by mixing of 1,4-butanediol monoacetate (91.79% purity, main impurity is 1,4-diacetate, 177.0 g) with CH₂Cl₂ (1350 mL, 1800 g). Feed-2 solution was prepared by mixing in a glass bottle at room temperature 100% nitric acid (85.2 g), 98% sulfuric acid (338.3 g) and water (24.45 g). The Feed-1 and Feed-2 solutions are then fed at 17.50 g/min and 4.23 g/min flowrate, respectively, to a Coming glass microreactor (internal volume 10 mL) with integrated heat-exchanger structures thermostated at 25 °C. The reactor outlet was quenched in water. The resulting biphasic system was stirred for 5 minutes, the aqueous phase discarded and the organic phase washed with the same weight of aqueous 5% NaHCO₃. Yield of acetic acid 4-nitryloxybutyl ester is about 93%.

**Comparative Example 1: Preparation of BDMN according to WO-A 2004/043898**

A stock solution of "stabilized" nitric acid was prepared by mixing fuming nitric acid (84.7 g), water (15.3 g) and urea (0.8 g) and stirring until gas evolution (N₂ and CO₂) stops. 56.7 g of the stock solution (0.76 mol HNO₃) and CH₂Cl₂ (100 mL) were loaded in a 250 mL reactor and cooled to 0 °C. A solution of 1,4-butanediol (5.16 g, 56 mmol) and CH₂Cl₂ (2.22 g) was added dropwise under vigorous stirring to the cold emulsion, while keeping the temperature around 0 °C (10 min). After 30 min, the reaction was quenched by adding 56.7 g of crushed ice. The excess acid was then neutralized with 40% NaOH (ca. 71 g) and the phases separated, affording 137.3 g of an organic mixture. Direct injection in GC (FID) gave BDMN 5.3 %-area, 1,4-butanediol dinitrate 1.2 %-area in CH₂Cl₂ (ad 100%) corresponding to a selectivity of about ∼82%. For further use, the BDMN comprising mixture must be purified according to patent WO-A 2004/043897. Due to safety regulations the reaction cannot be carried out in more concentrated solution.

## Claims

1. A method for the preparation of a compound having at least one nitryloxy group and at least one hydroxy group or an esterified hydroxy residue, said esterified hydroxy residue representing a hydroxy group esterified with an acid or an acid anhydride other than nitric acid and thus consisting of an acid moiety and the hydroxy oxygen atom of the hydroxy group, comprising reaction of a compound comprising at least one esterified hydroxy group and a least one free hydroxy group with nitric acid, optionally in the presence of one or more other acids and/or acid anhydrides, to obtain a compound wherein the at least one free hydroxy group is nitrated, and, optionally, subsequent hydrolysis or transesterification of the at least one esterified hydroxy residue.

2. The method of claim 1, wherein the acid moiety of the at least one esterified hydroxy residue is selected from acyl, aroyl, alkanesulfonyl and arenesulfonyl groups, said acyl or alkanesulfonyl groups consisting of an alkyl moiety and a carbonyl or sulfonyl group respectively, said alkyl moiety being optionally further substituted with one or more halogen atoms, and said aroyl and arenesulfonyl groups consisting of an aryl moiety and a carbonyl or sulfonyl group respectively, said aryl moiety being optionally further substituted with one or more halogen atoms and/or one or more nitro, alkyl, alkoxy, aryl and aryloxy groups.

3. The method of claims 1 or 2, wherein the hydrolysis is carried out chemically or enzymatically.

4. The method of any of claims 1 to 3, wherein the compound comprising at least one esterified hydroxy residue and a least one free hydroxy group is obtained by esterification of a compound having at least two free hydroxy groups.

5. The method of any of claims 1 to 4, wherein the compound comprising at least one esterified hydroxy residue and a least one hydroxy group is obtained by partial ester hydrolysis of a compound having at least two esterified hydroxy residues.

6. The method of any of claims 1 to 5, wherein the other acid or acid anhydride is selected from the group consisting of sulfuric acid, methanesulfonic acid, toluenesulfonic acid, alkanoic anhydrides and aroyl anhydrides.

7. The method of any of claims 1 to 6, wherein the reaction with nitric acid is carried out in a microreactor.

8. The method of claim 7, wherein the microreactor is equipped with one or more mixing zone(s).

9. A compound of formula
R¹C(O)O(CH₂)ₙONO₂ (IIa)
wherein R¹ is selected from the group consisting of C₂₋₁₈ alkyl and aryl, and wherein n is an integer from 2 to 18.

10. A compound of formula
R²C(O)O(CH₂)ₙONO₂ (IVa)
wherein R² is a halogen atom or a residue selected from the group consisting of O₂NO(CH₂)ₘO-, R³R⁴N- and R⁵S-, wherein R³, R⁴ and R⁵ independently are selected from the group consisting of C₁₋₁₈-alkyl and aryl groups, said C₁₋₁₈-alkyl groups optionally being further substituted with one or more halogen atoms, said aryl groups optionally being further substituted with one or more halogen atoms and/or one or more nitro, alkyl, alkoxy, aryl and aryloxy groups, and wherein n and m independently are an integer from 2 to 18, with the exception of compounds wherein R² is chlorine and n is an integer from 2 to 6.
